Europäisches Patentamt

European Patent Office

Office européen des brevets

(11) Veröffentlichungsnummer: **0 234 261**
**A1**

# (12) EUROPÄISCHE PATENTANMELDUNG

(21) Anmeldenummer: 87100727.4

(22) Anmeldetag: 20.01.87

(51) Int. Cl.⁴: **A61K 7/06**

(30) Priorität: 30.01.86 DE 3602746

(43) Veröffentlichungstag der Anmeldung:
02.09.87 Patentblatt 87/36

(84) Benannte Vertragsstaaten:
AT CH DE ES FR GB IT LI NL SE

(71) Anmelder: Wella Aktiengesellschaft
Berliner Allee 65
D-6100 Darmstadt(DE)

(72) Erfinder: Konrad, Eugen
Mecklenburger Strasse 101
D-6100 Darmstadt(DE)
Erfinder: Mager, Herbert
Beaumont 5
CH-1700 fribourg(CH)
Erfinder: Hoch, Dietrich
Ringstrasse 48
D-6102 Pfungstadt(DE)

(54) Haarbehandlungsmittel und Verfahren zur Verbesserung des Zustandes der Haare.

(57) Gegenstand der Erfindung sind Mittel sowie ein Verfahren zur Verbesserung des Zustandes der Haare auf der Basis einer synergistischen Kombination von Glycin mit einer physiologisch verträglichen, von Aminogruppen freien aliphatischen organischen Säure, insbesondere Zitronensäure, und einer Wachs-und/oder Ölkomponente, insbesondere einer Mischung von Vaseline und Fettalkoholen.

Die haarkonditionierend wirksame Kombination Glycin/physiologisch verträgliche, von Aminogruppen freie aliphatische organische Säure/Wachs-und/oder Ölkomponente ist physiologisch völlig unbedenklich und kann auch zusammen mit anionischen Tensiden eingesetzt werden. Die erfindungsgemäßen Mittel wirken entwirrend auf das Haar, glätten die Haaroberfläche und verbessern den Griff sowie die Kämmbarkeit des Haares.

## Haarbehandlungsmittel und Verfahren zur Verbesserung des Zustandes der Haare

Die Erfindung betrifft Haarbehandlungsmittel auf der Basis einer synergistischen Kombination aus Glycin, einer von Aminogruppen freien aliphatischen organischen Säure sowie einer Wachs-und/oder Ölkomponente. Gegenstand der Patentanmeldung ist ferner ein Verfahren zur Verbesserung des Zustandes der Haare.

Durch öfteres Bleichen, Dauerwellen und Färben, aber auch durch häufiges Waschen der Haare mit entfettenden Tensiden, kommt es zu einer Schädigung der Haarstruktur. Das Haar wird spröde, und es verliert seinen Glanz. Weiterhin lädt sich das Haar beim Kämmen elektrostatisch auf, und die aufgerauhte Haaroberfläche verursacht Verfilzungen sowie Verknotungen des Haares. Hierdurch wird das Kämmen sehr erschwert.

Haarbehandlungsmittel mit einer kämmbarkeitsverbessernden und pflegenden Wirkung haben daher eine erhebliche Bedeutung erlangt. Derartige Mittel werden beispielsweise häufig in Form einer klaren Haarpflegespülung oder auch in Emulsionsform, als sogenannte Creme-Rinses, nach der Haarwäsche im noch nassen Haar verteilt, einige Minuten bis eine Stunde einwirken gelassen und dann mit Wasser ausgespült.

Als Wirkstoffe zur Verbesserung der Haarstruktur werden hauptsächlich kationische Tenside, insbesondere quaternäre Ammoniumverbindungen, wie Cetyltrimethylammoniumchlorid, in Kombination mit verschiedenen wachsartigen Zusätzen, wie zum Beispiel Vaseline, Fettalkoholen und Fettsäureestern, eingesetzt.

Haarbehandlungsmittel auf der Basis der vorstehenden konditionierenden Wirkstoffe zeigen jedoch nur bei der Behandlung von trockenem und porösem Haar zufriedenstellende Ergebnisse. Für die Behandlung von rasch nachfettendem Haar sind sie weniger gut geeignet, da durch ihre Anwendung die natürliche Nachfettung der Haare noch verstärkt wird, wodurch sich wiederum die Haltbarkeit der Frisur verschlechtert.

Die Ursachen der starken Nachfettung der Haare sind zum einen die nach dem Ausspülen im Haar verbleibenden Reste des Haarbehandlungsmittels, zum anderen die in diesen Mitteln enthaltenen kationischen Emulgatoren. Die vom Haar absorbierten kationischen Emulgatoren bewirken eine Hydrophobierung der Haaroberfläche, wodurch sich die Absonderungen der Talgdrüsen rascher im Haar verteilen können. Außerdem lassen sich kationische Emulgatoren wegen ihrer Unverträglichkeit mit anionischen Tensiden in Haarbehandlungsmittel mit einem Gehalt an diesen Tensiden, wie zum Beispiel in viele Shampoos oder Haarfärbemittel, nicht einarbeiten.

Weiterhin ist zu bemerken, daß kationische Tenside in der Regel eine schlechte Haut-und Schleimhautverträglichkeit besitzen. Aus diesem Grund kann die Wirksamkeit der üblichen Creme-Rinses am Haar nur begrenzt durch Erhöhung der Tensidkonzentration gesteigert werden.

Es wurde bereits mehrfach versucht, die oben genannten Nachteile durch die Anwendung von Aminosäuren, wie zum Beispiel einer schwach sauren Mischung verschiedener Aminosäuren und Vitamine - (siehe US-PS 4 201 235), als haarkonditionierenden Bestandteil in Haarbehand lungsmitteln zu vermeiden. Die Herstellung dieser aus einer Vielzahl von verschiedenen Vitaminen und Aminosäuren bestehenden Mischungen ist jedoch sehr aufwendig und teuer.

Weiterhin ist aus der Literatur Cosmetics & Toiletries Vol. 98 (1983), Seite 59 bis 68 die Verwendung von Keratinhydrolysaten und Zitronensäure in einem "Neutralizing Shampoo" bekannt. Dieses Shampoo besitzt jedoch nur eine geringe pflegende Wirkung und führt zu einer starken Austrocknung der Haare. Aus diesem Grunde ist im Anschluß an die Haarwäsche eine, gegebenenfalls sogar mehrmalige, Nachbehandlung mit einem Haarkonditionierungsmittel erforderlich.

Ebenfalls ist aus der Literatur W. Fassbender, Parfümerie und Kosmetik 39 (1), Seite 11 bis 16 (1958) bekannt, daß Aminosäuresole, welche 18 bis 22 verschiedene Aminosäuren enthalten, in der Pharmazie und Kosmetik, zum Beispiel in schwach sauer eingestellten Hautbehandlungs-und Haarpflegemitteln, eingesetzt werden können. Die Herstellung dieser Aminosäuresole erfolgt durch fraktionierte Hydrolyse von natürlichen Proteinen und anschließende Reinigung der erhaltenen Hydrolysate. Es ist deshalb schwierig die für die Qualität von kosmetischen Mitteln wichtige gleichbleibende Zusammensetzung der Aminosäuresole zu gewährleisten.

Aufgabe der Erfindung ist es, ein Haarbehandlungsmittel und ein Haarbehandlungsverfahren auf der Basis besser geeigneter haarkonditionierender Wirkstoffe zur Verfügung zu stellen und dadurch die vorstehend geschilderten Nachteile zu vermeiden.

Hierzu wurde nun völlig überraschend gefunden, daß Haarbehandlungsmittel, dadurch gekennzeichnet, daß sie eine Kombination von

a) 0,5 bis 25 Gewichtsprozent, vorzugsweise 3,0 bis 15 Gewichtsprozent, Glycin,

2

b) 1,0 bis 10 Gewichtsprozent, vorzugsweise 0,5 bis 5,0 Gewichtsprozent, einer physiologisch verträglichen, von Aminogruppen freien aliphatischen organischen Säure und

c) 0,5 bis 10 Gewichtsprozent, vorzugsweise 1,0 bis 5,0 Gewichtsprozent, einer Wachs-und/oder Ölkomponente enthalten, die gestellte Aufgabe in hervorragender Weise erfüllen.

Eine besonders bevorzugte Ausführungsform der Erfindung ist eine Haarpflegeemulsion, welche eine Kombination von

a) 3,0 bis 15 Gewichtsprozent Glycin,

b) 0,5 bis 5,0 Gewichtsprozent Zitronensäure und

c) 1,0 bis 5,0 Gewichtsprozent einer 1:1 Mischung aus Vaseline und Fettalkoholen enthält.

Während bei Haarbehandlungsmitteln auf alleiniger Basis von Glycin oder auf alleiniger Basis einer physiologisch verträglichen, von Aminogruppen freien aliphatischen organischen Säure oder auf alleiniger Basis einer Wachs-und/oder Ölkomponente oder auf der Basis einer Kombination von zwei dieser drei in den erfindungsgemäßen Mitteln enthaltenen Komponenten keine zufriedenstellenden haarkonditionierenden Eigenschaften festzustellen sind, zeigen die erfindungsgemäßen Haarbehandlungsmittel auf der Basis einer synergistischen Kombination von Glycin mit einer physiologisch verträglichen, von Aminogruppen freie aliphatischen organischen Säure und einer Wachs-und/oder Ölkomponente eine ausgezeichnete kämmbarkeitsverbessernde Wirkung, ohne dabei das Haar zu belasten. Insbesondere wird durch diese Mittel die Naßkämmbarkeit der Haare wesentlich verbessert. Weiterhin wirken sie adstringierend und entwirrend auf das Haar, glätten die Haaroberfläche und verbessern den Griff des Haares.

Die guten haarkonditionierenden Eigenschaften der erfindungsgemäßen Mittel sind insbesondere deshalb überraschend, weil sie im Widerspruch stehen zu der in der Literatur W. Fassbender, Kosmetik und Parfümerie **39** (1), Seite 11 bis 16 (1958) vertretenen Meinung, wonach der Einsatz einzelner isolierter Aminosäuren in kosmetischen Präparaten zwecklos, wenn nicht gar schädlich ist.

Die erfindungsgemäßen Mittel sind frei von hautirritierenden kationischen Tensiden. Stattdessen wird Glycin, eine hautfreundliche, zellwachstumsfördernde Aminosäure, eingesetzt.

Beispiele für geeignete, in den hier beschriebenen Mitteln enthaltene physiologisch verträgliche, von Aminogruppen freie aliphatische organische Säuren sind insbesondere wasser-oder wasser-alkohollösliche aliphatische organische Säuren, wie zum Beispiel Milchsäure, Weinsäure, Pimelinsäure, Adipinsäure, Malonsäure, Bernsteinsäure, Glutarsäure, Glyoxylsäure und Zitronensäure, wobei letztere besonders bevorzugt ist.

Für den Einsatz in den erfindungsgemäßen Haarbehandlungsmitteln geeignete Wachskomponenten sind zum Beispiel Wollwachs (Adeps Lanae), Bienenwachs, höhere Fettalkohole, wie beispielsweise Cetyl-und Stearylalkohol, und Vaseline (ein Gemisch aus Paraffinen und Kohlenwasserstoffölen, welches bei 35 bis 45 Grad Celsius schmilzt), während als Ölkomponente beispielsweise Paraffinöl und Fettsäureester, wie zum Beispiel die Glycerinester von Palmitin-,Stearin-und Ölsäure, verwendet werden können.

Die Wachs-und Ölkomponenten können alleine oder im Gemisch miteinander in den erfindungsgemäßen Haarbehandlungsmitteln eingesetzt werden. Besonders vorteilhaft ist hierbei die Verwendung einer 1:1 Mischung von Vaseline und Fettalkoholen.

Die in der Anmeldung beschriebenen Haarbehandlungsmittel können in einer beliebigen für die Haarbehandlung geeigneten Zubereitungsform, wie zum Beispiel in Form einer Lotion, Emulsion oder eines Gels, vorliegen. Bevorzugte Zubereitungen sind Haarspülungen, Haarpflegeemulsionen, Haarkurpackungen, Haarfestiger oder Shampoos. Die erfindungsgemäßen Haarbehandlungsmittel können aber auch als Haarfärbemittel, Haartönungsmittel oder als Fixiermittel für die Haarverformung vorliegen.

Es handelt sich dabei um Zubereitungen, die je nach ihrem Anwendungszweck für kürzere oder längere Zeit auf dem Haar verbleiben.

Durch ihren Gehalt an der beschriebenen Wirkstoffkombination Glycin/physiologisch verträgliche, von Aminogruppen freie aliphatische organische Säure/Wachs-und/oder Ölkomponente wird gleichzeitig eine Konditionierung des behandelten Haares bewirkt. Besonders bevor zugt sind jedoch solche Zubereitungen, die hauptsächlich oder ausschließlich dem Ziel einer Verbesserung des Zustandes der Haarstruktur dienen.

Die Zusammensetzung dieser kosmetischen Zubereitungen stellt eine Mischung der konditionierend wirkenden Kombination aus Glycin, einer physiologisch verträglichen, von Aminogruppen freien aliphatischen organischen Säure sowie einer Wachs-und/oder Ölkomponente mit weiteren für Haarbehandlungsmittel üblichen Bestandteilen dar.

Als übliche Bestandteile von Haarbehandlungsmitteln kommen insbesondere Wasser, niedere aliphatische Alkohole, wie zum Beispiel Ethanol, Propanol und Isopropanol, mehrwertige Alkohole, wie Glycerin und Propylenglykol, anionische, amphotere oder nichtionogene Tenside, wie zum Beispiel Fettalkoholsulfate, Fettalkoholethersulfate, Alkylsulfonate, Alkylbenzolsulfonate, Fettsäuretauride, oxethylierte Fettalkohole, oxethylierte Alkylphenole, Fettsäurealkanolamide, weiterhin natürliche, modifizierte natürliche oder synthetische Polymere, wie zum Beispiel Schellack, Alginate, Gelatine, Pektine, Cellulosederivate, Chitosan, Polyvinylpyrrolidon, Polyvinylacetat, Acrylsäure-oder Methacrylsäurepolymerisate, basische Polymerisate von Estern aus Acrylsäure oder Methacrylsäure mit Aminoalkoholen beziehungsweise die Salze oder Quaternisierungsprodukte dieser basischen Polymerisate, Polyacrylnitril sowie Copolymerisate aus derartigen Verbindungen, wie beispielsweise Polyvinylpyrrolidon-Vinylacetat, ferner Verdicker, wie zum Beispiel Stärke und Cellulosederivate, sowie außerdem Pflegestoffe wie zum Beispiel Lanolinderivate, Cholesterin und Pantothensäure, weiterhin Farbstoffe, Pigmente, Parfümöle, Antioxidanten und Konservierungsmittel in Betracht.

Die in den erfindungsgemäßen Haarbehandlungsmitteln enthaltenen Glycinsalze wirken, auch bei der angegebenen relativ hohen Konzentration, nicht emulsionsfeindlich.-Vielmehr wurde überraschenderweise festgestellt, daß der Zusatz von Glycin und einer physiologisch verträglichen, von Aminogruppen freien aliphatischen organischen Säure, insbesondere Zitronensäure, viskositätssteigernd und für die Stabilität der Emulsionen fördernd wirkt.

Ein weiterer Vorteil dieser Haarbehandlungsmittel ist ihre gegenüber Mitteln auf der Basis von üblichen kationischen haarkonditionierenden Wirkstoffen, wie zum Beispiel Fettsäurealkyltrimethylammoniumsalzen, erheblich bessere Augen-und Hautverträglichkeit.

Die vorliegende Erfindung betrifft weiterhin ein Verfahren zur Verbesserung des Zustandes der Haare, welches dadurch gekennzeichnet ist, daß das Haar mit einer ausreichenden Menge, im allgemeinen etwa 25 bis 50 Gramm, eines vorstehend beschriebenen erfindungsgemäßen Haarbehandlungsmittels bei einer Temperatur von etwa 15 bis 60 Grad Celsius in Kontakt gebracht wird. Falls es sich nicht um ein Haarbehandlungsmittel handelt, welches dazu bestimmt ist, dauernd auf dem Haar zu verbleiben, wird das Haar anschließend mit warmem Wasser gespült und wie üblich weiterbehandelt.

Die nachstehenden Beispiele sollen den Erfindungsgegenstand näher erläutern.

Beispiele

### Beispiel 1    Haarspülung

| | |
|---|---|
| 10,00 g | Glycin |
| 5,00 g | Zitronensäure, wasserfrei |
| 1,20 g | Cetylstearylalkohol |
| 1,20 g | Vaseline |
| 0,60 g | Laurylalkohol, zweifach oxethyliert |
| 0,20 g | Salicylsäure |
| 0,15 g | Natriumcetylsulfat |
| 0,15 g | Natriumstearylsulfat |
| 0,50 g | Parfümöl |
| 81,00 g | Wasser |
| ——— | |
| 100,00 g | |

35 g der obigen Haarspülung werden nach der Haarwäsche auf dem handtuchtrockenen stark verfilzten, sehr porösen Haar verteilt. Bereits während des Auftragens der Haarspülung ist eine deutliche Entwirrung der Haare festzustellen. Nach einer kurzen Einwirkungszeit wird das Haar mit warmem Wasser gründlich gespült. Durch diese Behandlung wird ein sehr glatter, kosmetisch angenehmer Griff des Haares und eine sehr gute Naßkämmbarkeit erreicht.

4

Beispiel 2                    Haarpflegeemulsion

| | |
|---|---|
| 5,00 g | Glycin |
| 2,50 g | Zitronensäure, wasserfrei |
| 1,20 g | Cetylstearylalkohol |
| 1,20 g | Vaseline |
| 0,60 g | Laurylalkohol, zweifach oxethyliert |
| 0,50 g | Parfümöl |
| 0,20 g | Salicylsäure |
| 0,15 g | Natriumcetylsulfat |
| 0,15 g | Natriumstearylsulfat |
| 88,50 g | Wasser |

100,00 g

Die Anwendung dieser Haarpflegeemulsion erfolgt wie in Beispiel 1.

Das Haar ist deutlich weniger verfilzt und besser kämmbar. Die Wirksamkeit dieser Haarpflegeemulsion ist aufgrund des verringerten Gehaltes an Glycin und Zitronensäure etwas geringer als die der Haarspülung nach Beispiel 1, dennoch aber völlig ausreichend.

**Ansprüche**

1. Haarbehandlungsmittel, dadurch gekennzeichnet, daß es eine Kombination von
   a) 0,5 bis 25 Gewichtsprozent, vorzugsweise 3,0 bis 15 Gewichtsprozent, Glycin,
   b) 1,0 bis 10 Gewichtsprozent, vorzugsweise 0,5 bis 5,0 Gewichtsprozent, einer physiologisch verträglichen, von Aminogruppen freien aliphatischen organischen Säure und
   c) 0,5 bis 10 Gewichtsprozent, vorzugsweise 1,0 bis 5,0 Gewichtsprozent, einer Wachs-und/oder Ölkomponente enthält.

2. Haarbehandlungsmittel nach Anspruch 1, dadurch gekennzeichnet, daß die physiologisch verträgliche, von Aminogruppen freie aliphatische organische Säure ausgewählt ist aus Milchsäure, Weinsäure, Pimelinsäure, Adipinsäure, Malonsäure, Bernsteinsäure, Glutarsäure und Zitronensäure.

3. Haarbehandlungsmittel nach Anspruch 1 und 2, dadurch gekennzeichnet, daß es als Wachs-und/oder Ölkomponente ein 1:1 Gemisch aus Vaseline und Fettalkoholen enthält.

4. Haarbehandlungsmittel nach Anspruch 1 bis 3, dadurch gekennzeichnet, daß es eine Haarspülung, eine Haarpflegeemulsion, ein Haarfestiger, eine Haarkurpackung oder ein Shampoo ist.

5. Haarpflegeemulsion dadurch gekennzeichnet, daß sie
   a) 3,0 bis 15 Gewichtsprozent Glycin,
   b) 0,5 bis 5,0 Gewichtsprozent Zitronensäure

und

   c) 1,0 bis 5,0 Gewichtsprozent einer 1:1 Mischung aus Vaseline und Fettalkoholen

enthält.

6. Verfahren zur Verbesserung des Zustandes der Haare, dadurch gekennzeichnet, daß das Haar mit einem Haarbehandlungsmittel nach einem der Ansprüche 1 bis 5 bei einer Temperatur von 15 bis 60 Grad Celsius in Kontakt gebracht wird.

## EUROPÄISCHER RECHERCHENBERICHT

Europäisches
Patentamt

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-B-1 220 969 (E. KRÜGERMEYER & CO.) <br> * Patentansprüche; Beispiele * | 1 | A 61 K 7/06 |
| X | DE-B-2 901 452 (SCHWECKENDIEK) <br> * Beispiele * | 1,3 | |
| X | DE-A-2 703 964 (SCHWECKENDIEK) <br> * Beispiele 2-5 * | 1,3 | |
| X | EP-A-0 056 595 (WELLA AG) <br> * Patentansprüche; Seite 4, Zeilen 25-27; Seite 6, Zeilen 10-14,20-34; Beispiel 2 * | 1 | |

| RECHERCHIERTE SACHGEBIETE (Int. Cl.4) |
|---|
| A 61 K |

Der vorliegende Recherchenbericht wurde für alle Patentansprüche erstellt.

| Recherchenort <br> DEN HAAG | Abschlußdatum der Recherche <br> 29-04-1987 | Prüfer <br> WILLEKENS G.E.J. |
|---|---|---|